# EUROPÄISCHE PATENTSCHRIFT

(11) **EP 0 519 227 B1**
(45) Veröffentlichungstag und Bekanntmachung des Hinweises auf die Patenterteilung: **23.08.1995**
(21) Anmeldenummer: 92108642.7
(22) Anmeldetag: 22.05.1992
(51) Int. Cl.: A61F 2/30

(54) **Verfahren zur Herstellung eines Knochenimplantates**
Process for producing a bone implant
Procédé de fabrication d'un implant osseux

(30) Priorität: 15.06.1991 DE 4119773
(43) Veröffentlichungstag der Anmeldung: 23.12.1992
(73) Patentinhaber: MAN Ceramics GmbH, 94453 Deggendorf (DE)
(72) Erfinder: Siebels, Wolfgang, W-8360 Deggendorf (DE)

(56) Entgegenhaltungen:
- EP-A- 0 007 287
- EP-A- 0 442 256
- US-A- 4 406 023
- US-A- 4 454 612
- US-A- 4 495 664

## Beschreibung

Die Erfindung bezieht sich auf ein Verfahren zur Herstellung eines Knochenimplantates, wobei ein Metallkörper mit einem Faserflechtwerk überzogen wird.

Solche Implantate werden üblicherweise mit einem Knochenzement in einen menschlichen Knochen eingesetzt. Derartige Prothesen bestehen aus Stahl, einer Kobalt- oder Titan-Basislegierung.

Implantate aus Metall werden nicht derart vom Körper aufgenommen, daß sich ein Knochenwachstum um die Prothese einstellt. Deshalb wird in solchen Fällen die Verankerung des Implantats im Knochen mittels Knochenzement bewirkt. Durch starke Belastungen, wie es z. B. beim Schaft einer Hüftgelenkprothese der Fall ist, lockert sich das Implantat jedoch wieder.

Der Erfindung liegt die Aufgabe zugrunde, eine Abhilfe gegen eine frühzeitige Lockerung der implantierten Prothese zu schaffen.

Die Aufgabe wird erfindungsgemäß durch die Merkmale des Anspruchs 1 gelöst.

Die aus Metall hergestellte Prothese wird anschließend in einer Flechtmaschine mit einem Flechtwerk überzogen, bei dem die Fasern sich genau an die Oberflachenkontur anpassen und mittels eines biokompatiblen Harzes eine Klebverbindung mit dem Metall eingehen. Die geflochtenen Fasern bilden eine strukturierte Oberfläche, die das Anwachsen der Knochensubstanz fördern. Vorzugsweise bestehen die Fasern aus Kohlenstoffasern, die ebenfalls das Knochenwachstum begünstigen. Mit der erfindungsgemäßen Lösung ist es gelungen, die aus mechanischer Sicht günstigen Metallimplantate ohne Knochenzement und dauerhaft implantieren zu können.

Aus der US-A-4,495,664 ist zwar ein Knochenimplantat aus Metall bekannt, das mit einem Faserflechtwerk überzogen ist Jedoch ist das Faserflechtwerk von einer Schicht aus Copolymer umgeben, das in Verbindung mit dem heranwachsenden Knochenmaterial eine Kombination mit knochenähnlichen Eigenschaften bildet. Eine Lockerung des Metallimplantats ist damit jedoch u. U. nicht auszuschließen.

Die Erfindung hat den weiteren Vorteil, daß bereits bestehende Metallimplantate in einfacher Weise nach gerüstet werden können.

Für die formschlüssige Verbindung zwischen Fasern und Metallkörper werden die Fasern vor dem Flechtvorgang mit einer Matrix getränkt. Vorteilhaft ist es jedoch, wenn der Metallkörper mit einem Klebmaterial bestrichen und die Flechtfasern unimprägniert aufgebracht werden. Damit wird die Oberflächenstruktur des Flechtwerkes bzw. des Implantats für den Anwachsprozeß verstärkt.

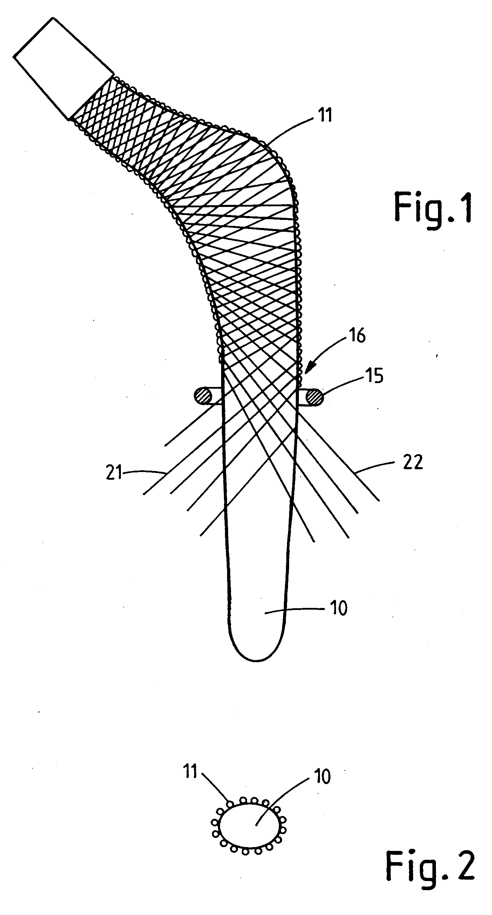

In der Zeichnung ist ein Ausführungsbeispiel der Erfindung schematisch dargestellt.

In der Zeichnung ist ein Metallschaft 10 einer Hüftgelenkprothese gezeigt, der in einer nicht dargestellten Flechtmaschine mit einem Flechtwerk 11 umspannt wird, wobei die Fasern 21, 22 in Flechtform auf die Oberfläche des Metallimplantats 10 gelegt werden. Im Flechtvorgang wird die Orientierung des Metallschaftes 10 verändert, und zwar derart, daß die Achse des im Fadenauge 15 der Flechtmaschine befindlichen Schaftbereiches 16 senkrecht zum Fadenauge gerichtet ist.

Das Flechtwerk 11 ist nach Aushärtung einer Matrix formschlüssig mit dem Metallimplantat 10 verbunden. Das Matrixmaterial kann entweder vorher auf die Metallkörperoberfläche aufgebracht oder durch Imprägnieren der Fasern 21, 22 mit dem Matrixmaterial eingebracht werden.

Als Fasern werden die körperverträglichen Kohlenstoffasern verwendet. Als Matrixmaterial dient beispielsweise Polyphenolglycidylether mit 4,4'-Diaminodiphenylsulfon.

Eine derartige Prothese kann ohne Knochenzement implantiert werden. Aufgrund der Materialien des Flechtwerkes und der strukturierten Oberfläche des Flechtwerkes 11 wird ein gutes Anwachsen und eine gute Knochenbildung um die Prothese 10 bewirkt, wodurch eine dauerhaft sitzende Prothese geschaffen ist. Altbestände von Metallimplantaten können außerdem in einfacher Art durch Überflechten nachgerüstet werden, so daß die Problematik des Anwachsens bzw. die vorzeitige Lockerung des Implantats vermieden wird. Zur Betonung der Rauhigkeit der Oberfläche ist es günstig, wenn das Matrixmaterial lediglich zur Klebeverbindung zwischen Metall und Fasern vor dem Flechtvorgang auf den Metallkörper aufgestrichen wird und nicht imprägnierte Fasern verwendet werden.

## Patentansprüche

1. Verfahren zur Herstellung eines Knochenimplantates, wobei ein Metallkörper (10) mit einem Faserflechtwerk (11) überzogen wird, dadurch gekennzeichnet, daß die Fasern (21, 22) direkt auf den Metallkörper (10) des Knochenimplantates aufgeflochten werden.

2. Verfahren nach Anspruch 1, dadurch gekennzeichnet, daß das Faserflechtwerk (11) mit dem Metallkörper (10) verklebt wird.

3. Verfahren nach Anspruch 1 oder 2, dadurch gekennzeichnet, daß das Faserflechtwerk (11) aus unimprägnierten Fasern (21, 22) hergestellt wird.

4. Verfahren nach einem der vorhergehenden Ansprüche, dadurch gekennzeichnet, daß das Faserflechtwerk (11) aus Kohlenstoffasern (21, 22) besteht.

## Claims

1. Process for producing a bone implant, wherein a braiding of fibres (11) is applied on a metal body (10), characterized in that the fibres (21,22) are braided directly on the metal body (10) of the bone implant.

2. Process according to Claim 1, characterized in that the braiding of fibres (11) is adhered to the metal body (10).

3. Process according to Claim 1 or 2, characterized in that the braiding (11) is produced using non-impregnated fibres (21,22).

4. Process according to one of the preceding Claims, characterized in that the braiding (11) is composed of carbon fibres (21,22).

## Revendications

1. Procédé de fabrication d'un implant osseux, comprenant un corps métallique (10) revêtu d'une tresse en fibres (11), caractérisé en ce que les fibres (21, 22) sont tressées directement sur le corps métallique (10) de l'implant.

2. Procédé selon la revendication 1, caractérisé en ce que la tresse en fibres (11) est collée sur le corps métallique (10).

3. Procédé selon la revendication 1 ou 2, caractérisé en ce que la tresse (11) est réalisée au moyen de fibres (21, 22) non imprégnée.

4. Procédé selon une des revendications précédentes, caractérisé en ce que la tresse (11) est faite de fibres de carbone (21, 22).
